# EUROPEAN PATENT APPLICATION

(11) **EP 2 111 875 A1**
(43) Date of publication of application: **28.10.2009**
(21) Application number: 08007976.7
(22) Date of filing: 25.04.2008
(51) Int. Cl.: A61L 9/03, A61L 9/12, A61L 9/14, A01M 1/20

(54) **Emanator for air treatment agent with odour detection**

(71) Applicant: Reckitt Benckiser (UK) Limited, Slough, Berkshire SL1 3UH (GB)
(72) Inventor: Belmonte, Elias c/o Reckitt Benckiser (UK) Limited, Hull, HU8 7DS (GB); Copeman, Matthew c/o Reckitt Benckiser (UK) Limited, Hull, HU8 7DS (GB); Walsh Steve c/o Reckitt Benckiser (UK) Limited, Hull, HU8 7DS (GB)
(74) Representative: Carlin, Robert George

(57) **Abstract**

An air treatment agent emanation device is described comprising a housing configured to receive a container of air treatment agent, odour detection means, emanation means adapted to emanate, in use, the air treatment agent, a controller in communication with said odour detection means and said emanation means, wherein the odour detection means comprises at least one humidity sensor, and wherein said odour detection means is operable, in use, to detect the level of humidity and/or odour and communicate the detected level to the controller. Methods of detecting odour and treating detected odour are also described.

## Description

### Field of the Invention

The present invention relates to an emanation device that is configured to emanate an air treatment agent into the surrounding environment based on a determination of the characteristics of the surrounding environment and particularly, but not exclusively, for the emanation of air treatment agents such as fragrances, deodorizing and/or pest control materials.

### Background

Devices are known in which a bottle of volatile liquid having an upwardly projecting wick can be accommodated wherein the device has a heater located in the vicinity of the upper end of the wick to accelerate the evaporation of volatile liquid from the wick. The bottle, wick and heater are retained within a housing of the device which also carries an electric plug. To operate the heater the device is plugged into a wall socket Devices of this type commonly claim to allow control of the rate of evaporation of the volatile liquids, for example, by varying the distance between the heater and the wick.

Devices are also known in which an aerosol air treatment agent, such as an air freshener, is held within an automatic spraying device. A powered mechanism periodically actuates the valve on the aerosol to emit a spray of the air treatment agent. Devices of this type commonly claim to allow control of the amount of sprayed air treatment agent over a fixed time period by the consumer being able to vary the time period between emissions. Such automatic spraying devices are typically unable to provide adjustment in response to external stimuli however.

In the field of automatic air freshening, different environments may require different responses from the device which are appropriate to the location of the device. For instance, when such a device is used in a domestic environment it would be desirable for the device placed in a room having large amount of thoroughfare, thus likely in more need of frequent fragrancing, to have more fragrance emanated. Conversely, when used in a room having only a small amount of thoroughfare, less frequent fragrancing would be desirable.

Whilst prior art devices are known to have user controls to alter the emanation rate to meet such a demand, this is not always satisfactory. For instance devices are known where a user may be able to manually change the emanation rate from a "normal" to "boost" mode, and then to switch it back to "normal" mode when this effect is no longer necessary (e.g. when the room is empty, or at night). Given the typical location of sources of electrical supply on walls (at a low level near the floor) or placement of electrical devices, this makes the switching process inefficient and inconvenient.

There is a need, therefore, for a device which overcomes some or all of the defects of the prior art.

### Summary of Invention

According to a first aspect of the present invention there is provided therefore an air treatment agent emanation device comprising:
a housing configured to receive a container of air treatment agent;
odour detection means;
emanation means adapted to emanate, in use, the air treatment agent;
a controller in communication with said odour detection means and said emanation means; wherein the odour detection means comprises at least one humidity sensor and
wherein said odour detection means is operable, in use, to detect the level of humidity and/or odour and communicate the detected level to the controller.

The use of at least one humidity sensor as or within the odour detection means is advantageous since the presence of odour is commonly accompanied by an increase in the levels of humidity. Currently pure odour detection technology is in its infancy and can be expensive to implement and is generally not capable of detecting across a comprehensive odour spectrum. A further drawback is that most commercially available odour sensors consume a considerable amount of power, thus, rendering them not inexpensive to operate and potentially unsuitable for use with a device which is battery powered rather than powered by mains electricity. Therefore detecting for changes in the level of humidity may provide a convenient, reliable and relatively inexpensive technique for detecting the presence of odour within the environment surrounding the device.

The odour detection means may be provided by at least one humidity sensor and at least one temperature sensor. In this arrangement at least two inputs on the levels of humidity and temperature respectively in the environment surrounding the device may be analysed by the odour detection means and/or controller. These at feast two inputs may be cross-referenced against each other to provide a more accurate determination as to the extent of odour in the surrounding environment

In an alternative arrangement the odour detection means may be provided by at least one humidity sensor and at least one odour sensor and, preferably by one humidity sensor and one odour sensor, In this arrangement at least two inputs on the levels of humidity and odour respectively in the environment surrounding the device may be analysed by the odour detection means and/or controller. These at least two inputs may be cross-referenced against each other to provide a more accurate determination as to the extent of odour in the surrounding environment

In a most preferred arrangement of the present invention however, the odour detection means is provided by at least one humidity sensor and at least one odour sensor and at least one temperature sensor. Ideally the odour detection means is provided by a humidity sensor and an odour sensor and a temperature sensor.

Preferably the emanation means is provided in the form of at least one heater means and/or at least one electric fan means when the container to be used with the device comprises a wick having a proximal end region within the container and a distal end region above the container from which the air treatment agent, in use of the device, is to be emanated.

Alternatively the emanation means may be selected from a component having properties suitable for emanating the particular air treatment agent to be used with the device into the surrounding environment. For instance, where the air treatment agent is in liquid form, the emanation means may be provided in the form of a nebuliser or the like or a piezo-electric emanation plate or the like.

As a further alternative, the emanation means may be provided in the form of an actuation means when the container to be used with the device comprises air treatment agent held under pressure within the container, said actuation means being operable to cause a valve in the container to be opened, thus permitting air treatment agent to be sprayed. The actuation means may take the form of an actuator arm or the like which is configured to make contact with the container to cause the valve to be opened. Alternatively, the actuation means may take the form of a solenoid valve or the like which may be activated to cause the release of the air treatment agent from the container.

The controller is preferably operative to provide an increased amount of emanation of air treatment agent in response to the inputfrom the odour detection means informing the controller of an increased amount of odour in the environment surrounding the device. The input from the sensor(s) to the odour detection means may be analysed by said means prior to said means communicating with the controller. Alternatively the sensor(s) of the odour detection means may communicate directly With the controller.

The odour detection means may be operable to detect common household odours (and the chemicals which constitute) these malodours. For example: kitchen malodour, bathroom malodour; tobacco smoke; pet odours; mould and/or mildew; body odour; fish; onions; garbage; fragrance from other products (such as detergents, polishes, cleaning products etc). To facilitate such detection the odour detection means may be operable to detect at least some of the following chemical components: amines and nitrogen compounds; acids and/or sulphur compounds, such as mercaptans, thioacids, thioesters, sulfides, phenols and skatole.

The devices of the present invention are typically intended for domestic use and the like, therefore, the surrounding environment referred to means within the context of the present invention in the same room, say within 50 m² of the device; and preferably within 40 m² of the device: and more preferably within 25 m² of the device; and most preferably within 15 m² of the device.

The device of the present invention may be provided with an indicator wherein said indicator is operable to indicate to a user what function the device is currently performing; such as whether the device has detected odour or not The indicator may be operable to provide a visual indication and/or provide an audible indication. Preferably the indicator is configured to provide a visual indication by emitting light from one or more light sources, preferably one or more LEDs. The one or more light sources may be adapted to emit a different colour of light to indicate the current function the device is performing. Additionally or alternatively, the one or more light sources may blink or flash to indicate the current function that the device is performing.

Alternatively or additionally, the device may be operable to visually indicate the function currently being performed via a screen. The screen may be an LCD screen that is adapted to provide a message to a user, for instance such messages could include "ON". "ODOUR DETECTED", "BOOSTING", "OFF", etc.

The device may be powered by mains-supplied electricity and/or be battery powered and/or be powered by solar cells located on the device. Most preferably the device is battery powered.

The housing may be configured to receive the container of air treatment agent by being suitably shaped to receive the container therein. Securing means may be provided within the housing or by the housing to secure the container within the housing until a user wishes to replace the container, such as when the container is spent of air treatment agent and a new container is to be used with the device.

The housing may substantial surround the container in order to better secure the container therein to improve the safety of the operation of the device during use.

The housing may be provided with one or more apertures therethrough wherein said aperture(s) are in registration with the odour detection means or at least a part of the odour detection means. This arrangement may improve the ability of the odour detection means to determine the conditions in the surrounding vicinity of the device. The one or more apertures may be located within a recess in the device as this may serve to normalize the detection operations of the odour detection means.

Various air treatment agents may be emanated by the device including but not limited to:
fragrances; pesticides; insecticides; bactericides; detergents; disinfectants; odour neutralizers. Preferably however, the air treatment agent is a fragrance and/or odour neutalizer.

According to a second aspect of the present invention there is provided therefore a method for detecting odour and treating detected odour comprising the steps of:
placing a device according to the first aspect of the present invention in an operational mode; using the odour detection means of the device to detect at least the humidity in the environment surrounding the device;
the device emanating an air treatment agent from a container holding said agent into the surrounding environment in response entirely or in part to said detected level of humidity.

The controller communicates with the emanation means in order to instruct the emanation means to emanate air treatment agent. The controller and/or odour detection means may determine whether the emanation of air treatment is required by analysing the input(s) thereto relative to a base level. This base level may be:
calculated by the device after being operated in a particular environment for a period of time; constantly calculated such that the device is able to modify the base level over time relative to the input it receives from the sensor means over time;
a pre-determined value that is stored by the controller; or
controlled by a user.

There may be more than one base level. One of said base levels may be selectable by a user depending on the environment in which they wish to use the device and/or the effect they wish to achieve.

In an alternative arrangement the base level may be a background level for the particular environment in which the device is located, the device being operable to calculate the background level against which a current level could be analysed to determine whether these levels are separated by more than a predetermined amount.

In this alternative arrangement the controller and/or odour detection means may be operable to calculate the current airborne agent level by calculating an average of a predetermined number of most recent readings of the detector. Preferably, two to five of the most recent readings, more preferably three of the most recent readings.

The controller and/or odour detection means may be operable to calculate the deviation of the current airborne agent level from the background level by means of a subtraction of one from the other and/or by means of a ratio of one to the other.

Preferably, a deviation is calculated by subtracting the background level from the current airborne agent level and dividing that amount by the background level value. The result may be multiplied by a constant, for ease of display and/or use.

The present invention preferably operates not by using a pre-defined value for the background airborne agent level, but rather by calculating the background level and using this calculated background level to control the release of the at least one air treatment agent. This arrangement may be advantageous as the device would then be operable to adapt how it releases the one or more air treatment agents depending on the characteristics of the surrounding environment in which it is used.

Preferably, the controller and/or odour detection means are operable to calculate the background airborne agent level by calculating an average of a longer time period than that over which the current airborne agent level is calculated.

Preferably, the background airborne agent level and the current airborne agent level are temporally offset, preferably by at least 5 seconds, more preferably by at least 10 second, more preferably by at least 20 seconds.

Once the device is placed into an operational mode, the background airborne agent level may be an average of the levels of airborne agent detected by the device throughout the duration of that operational mode. In this arrangement the device may better 'leam' the characteristics of its local environment and, during use, will be better able to provide for the release of an air treatment agent(s) when the current airborne agent level deviates from the background level by more than the predetermined amount If a user wishes to move the device to an alternative location, a user may be encouraged to disengage the device from the operational mode, this disengagement may have the effect of resetting the average levels of background agent such that the device is operable to 'learn' the characteristics of its new environment when placed back into the operational mode by calculating the average background agent level from no existing starting point

Preferably, the controller and/or odour detection means are operable to calculate the background airborne agent level by calculating an average of a predetermined number of some or all of the most recent readings of the detector. Preferably 10 to 10,000 of the most recent readings, more preferably 20 to 5,000 of the most recent readings, and most preferably 50 to 1000 of the most recent readings.

The device may be provided with an initial setting mode wherein when the device is first powered up, the controller and/or odour detection means will automatically calibrate based on the existing background odour when the device is first switched on.

In an alternative or additional arrangement the controller and/or odour detection means are preferably operable to calculate the background level based on calculating a series of averages from rolling windows of measurements from the detector. Each rolling window may be an average of between two and ten readings, preferably six readings. Preferably, the windows do not overlap. Preferably, the windows span a time period of between 5 and 30 minutes, preferably between 10 and 25 minutes, preferably between 15 and 20 minutes. There may be approximately 30 to 50 windows.

Preferably, the controller and/or odour detection means are operable to discard the oldest window when a new window becomes available, preferably taking into account an offset between the current and background levels.

Preferably, the controller and/or odour detection means are operable to adjust the predetermined level of deviation from the background level that results in air treatment agent being released. The predetermined level may be manually adjustable. The deviation may be a positive or negative deviation.

### Brief Description of the Drawings

Embodiments of the invention will now be described, by way of example only, with reference to the following drawings in which:
Fig.1 illustrates a perspective view of a device of the present invention;
Fig.2 illustrates a sectioned side view of a device of the present invention;
Fig.3 illustrates a front elevation of the device with a front cover removed; and
Fig.4 illustrates a front elevation of the device with the front cover in place.

### Description of an Embodiment

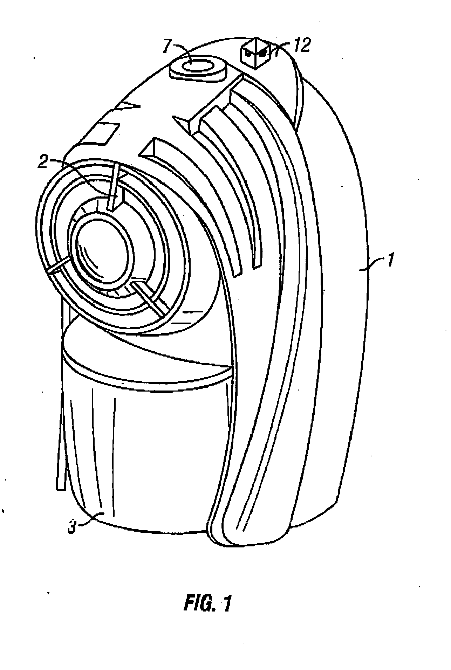

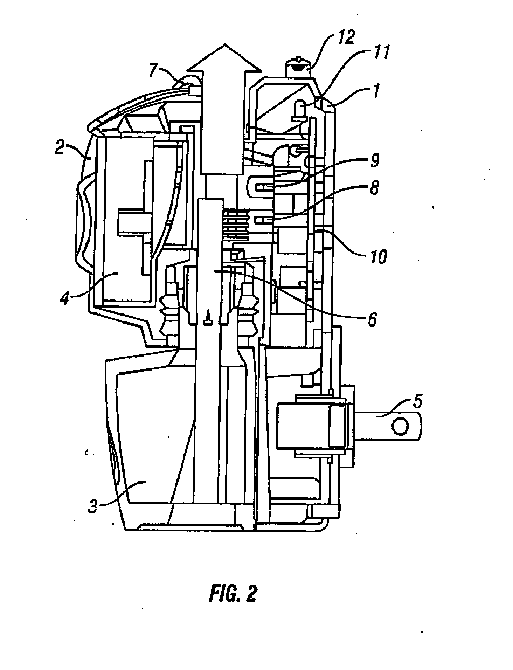

In general terms, the device 1 illustrated in Figs. 1 & 2 is shown with a container of volatile liquid engaged therewith. The container has a reservoir portion in the form of a glass bottle containing an air treatment agent in the form of a volatile liquid 3 and a wick 6 extending into the bottle. The wick 6 also extends above the top of the bottle through a seal and into a chimney means of the device 1. The wick 6 may be substantially cylindrical. The seal is present to retain the liquid 3 within the bottle should the device 1 be knocked over and/or inverted when the container is engaged therewith.

The device 1 has a housing 2 which partially extends over the container and its upper part. From the rear wall of the housing extends electrical plug formations 5.

The top of the housing 2 has a generally circular central aperture which defines the upper aperture of the chimney means. The upper aperture is aligned with a co-axial lower aperture (not shown), thus defining a channel therebetween for volatilised liquid to flow up and out of the upper aperture into the environment surrounding the device 1.

A first electrical heating means 8 and a second electrical heating means 9, if present, may be provided in the form of separate resistors, and preferably as positive temperature coefficient (PTC) therm istors and/or metal oxide resistors. However, either of both of the first or second electrical heating means 8.9 could be provided by way of a ring heater or the like, or a combination thereof.

An electrical fan 4 may also be provided. All of the fan 4 and the first and second electrical heating means 8,9 are in operable communication, either directly or indirectly, with an odour detection means provided in the form of a humidity sensor 12.

The humidity sensor 12 has openings therein to permit air surrounding the device to enter the sensor to permit the detection of the level of humidity in the surrounding environment. Although not illustrated, the humidity sensor 12 may have an odour sensor and/or temperature sensor located within or adjacent thereto.

Indirect operable communication may be via a controller (not shown) which could act as the principal receiver of information from the humidity sensor 12, process the provided information and direct the control of the aforementioned components.

Although not illustrated, the humidity sensor 12 may be provided behind a grid or the like having numerous apertures therethrough to permit the surrounding air to move toward the sensor. Preferably the grid would be located on, or protrudes through, an upper portion of the housing 2 in a position at least somewhat removed or remote from the aperture of the chimney means. This positioning of the grid may serve to prevent air treatment agent erroneously coming into contact with the grid, thus reducing erroneous humidity levels being detected by the sensor 12.

An example of one mode of operation of the device and the inter-relation of the components will now be explained.

The device 1 must first be placed in an operational mode. There may be a user-activated switch (not shown) to permit the device to be switched into the operational mode. The device 10 will draw power from the power source which is depicted as plug formations 5 to draw mains electric power, this could be from solar cells mounted on the device and/or one or more batteries however.

Initiating the operational mode will cause the first heater means 8 to warm up to a temperature that will cause the evaporation of the volatile liquid from the device 1. In the absence of the detection mode and/or humidity being detected, the first heater means 8 will continue to impart heat toward the wick for a period of time t₁ and, at the end of t₁ the heater means 8 will go into a rest period for time x where the heater means 8 will not draw any power.

A user controlled switch (not shown) may be provided to allow a user to adjust the value of t₁ and/or x.

However, the humidity sensor 12 may also draw power, either constantly or periodically, in order to sense for humidity in the vicinity of the device 1. If the humidity sensor 12 senses humidity it may be operable to communicate this information to the controller (not shown) which may be capable of interpreting this information as a level of odour in the vicinity of the device 1. Once the controller has received this information it may be operable to instruct the activation of at least one of: the second heater means 9; the fan 4; and/or a range of higher temperature in the first heater means 8; thus causing an increase in the rate of evaporation of the volatile liquid for a time interval of t₂. At the end of time interval t₂ the at least one of: the second heater means 9; the fan 4; and/or a range of higher temperature in the first heater means 8 will go into a rest period for time interval y where said means' and/or fan will not draw any power.

Subsequent detection of humidity by the humidity sensor 12 may also be communicated to the controller which will cause the activation of the at least one of; the second heater means 9; the fan 4; and/or a range of higher temperature in the first heater means 8 means only if time period y has elapsed.

When the device is first placed in an operational mode the controller may cause the activation to occur substantially immediately or after a short delay, say after 2 minutes.

Alternatively the controller may, after the first heater means 8 has been activated following being placed in an operational mode and on being informed of detection of humidity within the vicinity of the device, delay causing the activation of the at least one of: the second heater means 9; the fan 4; and/or a range of higher temperature in the first heater means 8, until a predetermined interval of time t₃ has elapsed. Once time period t₃ has elapsed subsequent activation following the detection of humidity would take place at a time interval of t₂.

Alternatively, when the operational mode is initiated the first heater means 8 will warm up to a temperature that will cause evaporation of the volatile liquid and this heater means will remain activated without rest period x.

The device may be manually or automatically switchable between a normal mode and a detection mode. Such automatic switching may be controlled by a timing mechanism and/or a sensor operably connected to the controller, such as a light sensor and/or sound detection means. The automatic switching may permit the device to consume less power by only permitting the device to operate in the detection mode of a limited period of time, thus conserving the power consumed by the humidity sensor 12. Such conservation of power being particularly useful where the device is powered by batteries and/or solar cell(s).

The illustrated device 1 is shown having an indicator 11 which is provided in the form of an LED. The LED(s) may be operable to provide a visual indication of the function currently being performed by the device. For instance, the LED could indicate when the device is in an operational mode by emitting a constant light which is converted to a flashing operation when humidity has been detected.

The indicator 11 may also be provided with an audio component (not shown) wherein this component is capable of giving an audible alert when a particular function is being performed and/or humidity has been detected or the like.

Alternatively or additionally, a screen (such as an LCD screen) could be presented on a prominent part of the device 1 to provide a message to a user indicating the current functioning of the device 1. For instance such messages could include "ON", "SENSING', "ODOUR DETECTED", "RESTING', "NORMAL MODE", "DETECTION MODE". "OFF".

The above description describes an embodiment comprising a controller, however, where the controller is not present the components may be inter-connected to be operably communicative with each other to implement the above-mentioned operational relationship.

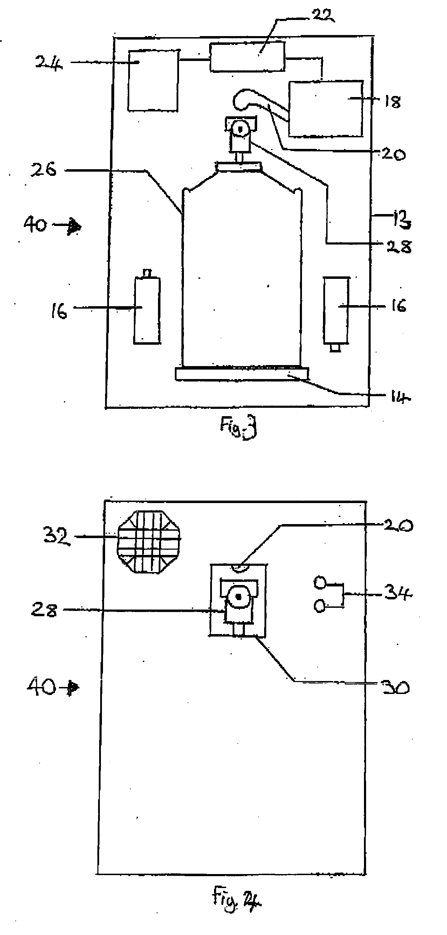

As shown in Fig. 3. a spraying device 40 comprises a housing 13 which supports a platform 14. The platform 14, in turn, is shaped to support and retain a container of air treatment agent 26, such as an aerosol of air treatment agent, when the device is in use. The housing 12 also supports an actuation means 18, a controller 22, a humidity sensor 24 and a power source which in Fig. 1 is depicted as a pair of batteries 16, although more or less batteries may be used. The humidity sensor 24 has openings therein to permit air surrounding the device to enter the sensor to permit the detection of the level of humidity in the surrounding environment Although not illustrated, the humidity sensor 24 may have an odour sensor and/or temperature sensor located within or adjacent thereto.

The device 40 is illustrated with a container of air treatment agent 26 loaded therein and, specifically, Fig. 3 is depicted with an aerosol of air treatment agent loaded therein. The air treatment agent is sprayed from the can and arm 20 connected to the actuation means 18 being moved in a downward direction and into contact with a spray head 28 of the aerosol. The movement of the arm 20 continues until the spray head is depressed and the valve within the aerosol is opened, thus, causing a quantity of air treatment agent to be sprayed therefrom. Preferably the device 40 has a metered dose aerosol loaded therein. A metered dose aerosol being advantageous as a single depression of the spray head will release a predefined quantity of air treatment agent from the aerosol regardless of the duration of time the spray head is depressed. However, a non-metered dose aerosol may be used in the device 40 as could a non-pressurised container possessing a pump mechanism to spray the air treatment agent therefrom.

Alternatively, the actuation means 18 Could take the form of a valve system, such as a solenoid valve system. Such a solenoid valve system may work together with a pressurised aerosol engaged therewith. Rather than initiate actuation by movement, the solenoid valve would be energised to initiate the release of a quantity of air treatment agent from the aerosol.

Regardless of the specific form of the actuation means 18, the mode of operation of the device and the inter-relation of the components will now be explained in detail.

The device 40 must first be placed in an operational mode. There will be a user-activated switch (not shown) to permit the device to be switched into the operational mode. The device 40 will draw power from the power source which may be mains electric power and/or solar cells mounted on the device, but is depicted in Fig. 1 as a pair of batteries 16.

Initiating the operational mode will cause the immediate spraying of air treatment agent, as described above. Thereafter further sprays of air treatment agent will periodically occur at time intervals of t₁. A user controlled switch (not shown) may be provided to allow a user to adjust the value of t₁.

However, the humidity sensor 24 may also draw power from the batteries 16, either constantly or periodically, in order to sense for humidity in the vicinity of the device 40. If the humidity sensor 24 senses humidity it is operable to communicate this information to the controller 22 which may be capable of interpreting this information as a level of odour in the vicinity of the device 40. Once the controller 22 has received this information it is operable to instruct the actuation means 18 to actuate again, thus causing a further spray of air treatment agent. Subsequent detection of humidity by the humidity sensor 24 may also be communicated to the controller which will cause the actuation means to actuate at a time interval of t₂ after the previous actuation, subject to t₂ ≤ t₁.

When the device is first placed in an operational mode the controller 22 may cause the actuation means 18 to actuate substantially immediately or after a short delay, say after 2-20 seconds, to cause a quantity of air treatment agent to be sprayed. Should humidity be detected immediately after the air treatment agent has been sprayed the controller 22 may cause the actuation means 18 to actuate again with subsequent actuations taking place at the time interval t₂.

Alternatively the controller 22 may, after the first actuation following being placed in an operational mode and on being informed of detection of humidity within the vicinity of the device, delay causing the actuator 18 to actuate until a predetermined interval of time t₃ has elapsed. Once time period t₃ has elapsed subsequent actuation following the detection of humidity would take place at a time interval of t₂.

The device 40 may be switchable between a normal mode and a detection mode, a user controllable switch (not shown) may be provided. In this embodiment the normal mode of operation permits the actuator 18 to actuate at the routine time interval of t₁. When the device is switched into detection mode however, the humidity sensor 24 may be operable to detect humidity in the vicinity of the device. Should humidity be detected, this information is communicated to the controller 22 which causes the actuation means 28 to actuate. Whilst the device remains in detection mode subsequent detection of humidity by the humidity sensor 24 is communicated to the controller 22 which causes the actuation means 18 to actuate at a time interval of t₂ wherein t₂ ≤t₁.

The device may be automatically switchable between the normal mode and the detection mode. Such automatic switching may be controlled by a timing mechanism and/or a sensor operably connected to the controller, such as a light sensor and/or sound detection means. The automatic switching may permit the device to consume less power by only permitting the device to operate in the detection mode of a limited period of time, thus conserving the power consumed by the humidity sensor 24. Such conservation of power being particularly useful where the device is power by batteries 16 and or solar cell(s).

Turning to Fig. 4, the housing may be shaped such that it is capable of substantially completely surrounding a container of air treatment agent 26. The front cover of the housing is illustrated and it can be seen that the front cover includes an aperture 30 therethrough which is in registration with the spray head 28 of the container 26. When the actuation means 18 cause the spraying of the air treatment agent, the air treatment agent exits the housing through the aperture 30 into the environment surrounding the device 40.

The humidity sensor 24 has a grid 32 with numerous apertures therethrough to permit surrounding air to move toward the sensor 24. The grid 32 is located on, or protrudes through, a portion of the front cover of the housing in a position remote from the aperture 30. This ensures that the grid 32 is spaced away from the aperture which may prevent sprayed air treatment agent erroneously coming into contact with the grid 32, thus reducing erroneous humidity levels being detected by the sensor 24.

Although not illustrated, the humidity sensor 24 may have an odour sensor and/or temperature sensor located within or adjacent thereto.

Fig. 4 further illustrates an indicator 34 which is provided in the form of two LEDs. The LEDs may be operable to provide a visual indication of the function currently being performed by the device. For instance, one LED could indicate when the device is in an operational mode and the other LED could indicate when humidity has been detected.

The indicator 34 may also be provided with an audio component (not shown) wherein this component is capable of giving an audible alert when a particular function is being performed and/or humidity has been detected or the like.

Alternatively or additionally, the indicator 34 may be provided with an LCD screen (not shown) where the screen is adapted to provide a message to a user indicating the current functioning of the device 10. For instance such messages could include "ON", "SENSING", "ODOUR DETECTED", "RESTING", "NORMAL MODE", "DETECTION MODE", "OFF".

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. An air treatment agent emanation device comprising;
a housing configured to receive a container of air treatment agent;
odour detection means;
emanation means adapted to emanate, in use, the air treatment agent;
a controller in communication with said odour detection means and said emanation means; wherein the odour detection means comprises at least one humidity sensor; and
wherein said odour detection means is operable, in use, to detect the level of humidity and/or odour and communicate the detected level to the controller.

2. A device according to claim 1, wherein the odour detection means is provided by at least one humidity sensor and at least one temperature sensor.

3. A device according to claim 1, wherein the odour detection means is provided by at least one humidity sensor and at least one odour sensor.

4. A device according to claim 1, wherein the odour detection means is provided by at least one humidity sensor and at least one odour sensor and at least one temperature sensor.

5. A device according to any preceding claim, wherein the emanation means is provided in the form of at least one heater means and/or at least one electric fan means when the container to be used with the device comprises a wick having a proximal end region within the container and a distal end region above the container from which the air treatment agent, in use of the device, is to be emanated.

6. A device according to any of claims 1-4, wherein the emanation means is provided in the form of an actuation means when the container to be used with the device comprises air treatment agent held under pressure within the container, said actuation means being operable to cause a valve in the container to be opened, thus permitting air treatment agent to be sprayed.

7. A device according to any preceding claim, wherein the controller is operative to provide an increased amount of emanation of air treatment agent in response to the input from the odour detection means informing the controller of an increased amount of odour in the environment surrounding the device.

8. A device according to any preceding claim, wherein the odour detection means is operable to detect common household odours (and the chemicals which constitute) these malodours, including: kitchen malodour; bathroom malodour; tobacco smoke; pet odours; mould and/or mildew; body odour; fish; onions; garbage; fragrance from other products (such as detergents, polishes, cleaning products etc).

9. A device according to any preceding claim, wherein the odour detection means is operable to detect one or more of the following chemical components: amines and nitrogen compounds; acids and/or sulphur compounds, such as mercaptans, thioacids, thioesters, sulfides, phenols and skatole.

10. A device according to any preceding claim, wherein the device is provided with an indicator wherein said indicator is operable to indicate to a user what function the device is currently performing.

11. A device according to any preceding claim, wherein the device is battery powered and/or be powered by solar cells located on the device.

12. A device according to any preceding claim, wherein the housing is shaped to substantially surround the container.

13. A device according to any preceding claim, wherein the air treatment agent(s) to be emanated by the device include at least one of; fragrances; pesticides; insecticides; bactericides; detergents; disinfectants; odour neutralizers.

14. A method for detecting odour and treating detected odour comprising the steps of; placing a device according to any preceding claim in an operational mode:
using the odour detection means of the device to detect at least the humidity in the environment surrounding the device;
the device emanating an air treatment agent from a container holding said agent into the surrounding environment in response entirely or in part to said detected level of humidity.

15. A method according to claim 14, wherein the controller and/or odour detection means determines whether the emanation of air treatment is required by analysing the input(s) thereto relative to a base level.

16. A method according to claim 15, wherein the base level is:
calculated by the device after being operated in a particular environment for a period of time; or constantly calculated such that the device is able to modify the base level over time relative to the input it receives from the sensor means over time; or
a pre-determined value that is stored by the controller; or
controlled by a user.

17. A method according to claim 15, wherein there is more than one base level.

18. A method according to claim 17, wherein one of sold base levels is selectable by a user.
